# EUROPÄISCHE PATENTSCHRIFT

(11) **EP 0 702 087 B1**
(45) Veröffentlichungstag und Bekanntmachung des Hinweises auf die Patenterteilung: **19.01.2000**
(21) Anmeldenummer: 95114474.0
(22) Anmeldetag: 14.09.1995
(51) Int. Cl.: C12Q 1/18

(54) **Testmedium für einen mikrobiologischen Hemmstofftest und Verfahren zu seiner Durchführung**
Test medium for a microbiologic inhibitor substance test and process for carrying out the test
Moyen d'épreuve pour une épreuve microbiologique de substances inhibitrices et procédé de la mise en oeuvre de cette épreuve

(30) Priorität: 14.09.1994 DE 4432771
(43) Veröffentlichungstag der Anmeldung: 20.03.1996
(73) Patentinhaber: Landesvereinigung der Bayerischen Milchwirtschaft e.V., 80336 München (DE)
(72) Erfinder: Adriany, Ansgar, D-80997 München (DE)
(74) Vertreter: Pätzold, Herbert, Dr.-Ing.

(56) Entgegenhaltungen:
- EP-A- 0 005 891
- EP-A- 0 322 591
- CA-A- 2 056 581
- DE-A- 3 613 794
- US-A- 5 354 663

## Beschreibung

Die Erfindung bezieht sich auf ein Testmedium für einen mikrobiologischen Hemmstofftest gemäß dem Oberbegriff des Anspruches 1 und ein Verfahren zur Durchführung eines solchen Testes.

Testmedien der vorstehenden Art werden z. B. zum mikrobiologischen Nachweis von Hemmstoffen in Produkten vor allem tierischen Ursprungs benötigt. Das Testmedium enthält grundsätzlich einen Testorganismus (Testkeim), ein festes oder flüssiges Nährmedium und gegebenenfalls ein optisches Anzeigemittel (Indikator), welches durch den im Nährmedium wachsenden Testkeim einen Farbumschlag erfährt, wenn er hieran nicht durch wenigstens einen Hemmstoff aus der zu untersuchenden Probe gehindert wird, d. h. die für den Farbumschlag notwendige Vermehrung des Testkeims durch Hemmstoffe in der zu untersuchenden Probe gehemmt wird. Dabei sind Hemmstoffe originäre oder nicht originäre Stoffe, die den Testkeim hemmen. Zu den Hemmstoffen zählen auch Tierarzneimittelrückstände.

Unter Tierarzneimittelrückstände werden Rückstände pharmakologisch wirksamer Stoffe verstanden, die in Tierarzneimitteln für zur Lebensmittelerzeugung genutzte Tiere verwendet werden. Bei diesen Stoffen handelt es sich z. B. um Antibiotika, Sulfonamide und andere Chemotherapeutika.

Vorzugsweise bezieht sich die Erfindung auf ein Testmedium zum Nachweis von Tierarzneimittelrückständen in Milch und Milchprodukten. Die Erfindung ist jedoch hierauf nicht beschränkt. Die ihr zugrunde liegende Lehre ist grundsätzlich auch für die Untersuchung anderer Nahrungsmittel, Futtermittel und tierischer oder menschlicher biologischer Flüssigkeiten, z. B. Serum, Harn und Speichel anwendbar.

Zum Nachweis von Hemmstoffen in Milch ist der sogenannte Brillantschwarz-Reduktionstest bekannt (Kraack, J. und Tolle A., Milchwissenschaft 22 (1967), 669 - 673). Hierbei wird die zu testende Milchprobe in ein Reaktionssystem pipettiert, das einen Testorganismus (Testkeim), ein Nährmedium für den Testkeim und einen Indikator enthält. Bei dem Testorganismus handelt es sich um Bacillus stearothermophilus var. calidolactis, Stamm C 953. Als Nährmedium wurde zunächst der "Plate-Count-Agar" der Firma Difco verwendet. Später wurde auch das Antibiotic Medium No. 1 der Firma E. Merck und als Indikator Brillantschwarz verwendet. Die Probe enthält Hemmstoffe, wenn sie das Wachstum des Testorganismus hemmen und dadurch der blaue Indikator nicht in die gelbe Reduktionsstufe überführt wird.

Bekannt ist der Brillantschwarz-Reduktionstest als amtliche Methode zum Nachweis von Hemmstoffen in Rohmilch, pastenrisierter Konsummilch und Säuglings- und Kleinkindernahrung auf Milchbasis (Amtliche Sammlung von Untersuchungsverfahren nach § 35 LMBG, März 1987 und Dezember 1990, L01.00/11). Der verwendete Testkeim Bacillus stearothermophilus var. calidolactis weist eine besondere Empfindlichkeit gegenüber Penicillinen auf. Durch die Zugabe von Trimethoprim (Gudding, R., Acta Vet. Scand. 17(1976), 458-464 und EP-Patent 0 005 891) oder Tetroxoprim (DE-Patent 36 13 794) wird die Nachweisempfindlichkeit der Sulfonamide erhöht. Trimethoprim und Tetroxoprim wirken dabei als Hemmstoffwirkungsverstärker. Hier wird der synergistische Effekt dieser Stoffe mit Sulfonamiden ausgenutzt, wie er zur Therapie bakterieller Infektionen bekannt ist.

Nachteilig ist bei den bekannten Testverfahren, daß die in der EU-Verordnung Nr. 2377/90 für ein Gemeinschaftsverfahren zur Festsetzung von Höchstmengen für Tierarzneimittelrückstände in Nahrungsmitteln tierischen Ursprungs (zuletzt ergänzt durch 1430/94, Abl. Nr. L 156/6 vom 23.06.1994) festgelegten Höchstmengen (MRL-Werte) z. B. für Sulfonamide und Tetracycline in einem Testverfahren bisher nicht erfaßt werden konnten.

Aufgabe der Erfindung ist es, ein Testmedium für einen mikrobiologischen Hemmstofftest der eingangs genannten Art sowie ein Verfahren zum Nachweis von Hemmstoffen mittels eines solchen Testmediums anzugeben, mit dem bei kurzen Gesamttestzeiten (2,5 bis 3,5 h) und guter visueller Ablesbarkeit der Testergebnisse sowie ausreichend langer Lagerfähigkeit des Testmediums verbesserte Testergebnisse zu relativ kostengünstigen Bedingungen erzielt werden können. Das soll insbesondere für Tierarzneimittelrückstände in Lebensmitteln und Futtermitteln tierischen Ursprungs, vor allem in Milch und Milchprodukten gelten, wobei die dort bereits erzielten niedrigen Nachweisgrenzen z. B. für Penicilline unverändert erreichbar sein sollen, zusätzlich aber auch die Erfassung von amtlich festgelegten Höchstmengen für andere erzielbar sein sollen.

Ziel der Erfindung ist es, neue praktikable Wege für mikrobiologische Hemmstoffteste aufzuzeigen, die auch den Nachweis von Hemmstoffen erlauben, die bisher nicht oder nicht in befriedigender Weise in einem Test nachweisbar waren. Hierbei soll die Erfindung auch nicht auf die Verwendung eines bestimmten Testkeimes bzw. dessen Sporen beschränkt sein.

Die Aufgabe wird erfindungsgemäß mit den kennzeichnenden Merkmalen des Anspruches 1 gelöst.

Durch die CA-A-2 056 581 ist ein Testmedium der eingangs genannten Art bekannt, bei dem nur eine Indikatorart zugesetzt ist, die jeweils auf dem gleichen Wirkungsprinzip beruht.

Die Vorteile, die erst mit einem erfindungsgemäßen Testmedium erzielbar sind, das mindestens zwei Indikatoren mit unterschiedlichen Wirkungsprinzipien enthält, sind mit dem bekannten Testmedium nicht erzielbar.

Bei dem noch bekannten Testmedium der eingangs genannten Art nach der EP-A-0 322 591 wird ein Testmedium verwendet, das einen Indikator aufweist, der auf einem bestimmten Wirkungsprinzip beruht. Zusätzlich wird noch ein Elektronenübertrager (electron carrier) verwendet, der lediglich die Empfindlichkeit des Indikators verbessert, ohne daß damit die vorteile eines erfindungsgemäßen Testmediums erzielt werden, das wenigstens zwei Indikatoren mit unterschiedlichen Wirkungsprinzipien enthält.

Vorteilhafte Ausführungen und Weiterbildungen nach der Erfindung ergeben sich aus den Merkmalen der Unteransprüche und der nachstehenden Beschreibung lediglich für Beispiele von erfindungsgemäßen Testmedien und Verfahren zum Nachweis von Hemmstoffen mit diesen Testmedien, ohne daß die Erfindung hierauf beschränkt ist. So bieten sich dem Fachmann nach Kenntnis der Erfindung wirkungsgleiche oder wirkungsähnliche Abwandlungen ohne weiteres an, die im Rahmen der Erfindung liegen. Das gilt besonders für die Auswahl der Testkeime bzw. Sporen und die zur Anwendung kommenden Nährmedien, Indikatoren und Komplexbildner.

Anstelle des erfindungsgemäß vorgeschlagenen Komplexbildners EGTA Bis(aminoethyl)-glycolether-N,N,N',N'-tetraessigsäure (C₁₄H₂₄N₂O₁₀; M = 380,35 g/Mol; Schmelzpunkt 250 - 253 °C) z. B. zur Hemmstoff-Wirkungsverstärkung von Tetracyclinen und zur Germinationsbeschleunigung von Sporen der verwendeten Testkeime kann erfindungsgemäß auch wenigstens eine der nachstehenden Substanzen DTPA (3-Aza-3-(carboxymethyl)-pentamethylen-dinitrilotetraessigsäure), CDTA (1,2-Cyclohexylendinitrilotetraessigsäure), EDTA (Ethylendiamintetraessigsäure), NTA (Tri(carboxymethyl)amin), TTHA (Triethylentetraminhexaessigsäure) und N-(2-Hydroxyethyl)-ethylendiamin-N,N,N-triessigsäure (Laborhandbuch der Firma E. Merck: "Komplexometrische Bestimmungsmethoden mit Titriplex") verwendet werden. Die Komplexbildner besitzen jeweils eine hohe Bindungskapazität für die Komplexierung von Metallionen, die die Wirkung von Tierarzneimitteln und/oder die Keimung von Sporen beeinträchtigen.

Es ist vorteilhaft, wenn das Nährmedium frei oder weitgehend frei ist von Sulfonamid- und Pyrimethamin-Antagonisten. Vorteilhafterweise wird ein standardisierter Mueller-Hinton-Nährboden, z. B. der Firma Oxoid (Art.-Nr. CM337, Oxoid-Handbuch, 5. aktualisierte deutsche Ausgabe, Unipath GmbH, 46483 Wesel, Deutschland) verwendet. Es kann aber auch der ISO-Sensitest-Agar von Oxoid (Art.-Nr. CM 471) oder der DST-Agar (Diagnostic Sensitivity Test Agar) von Oxoid (Art-Nr. CM 261) oder auch der Isotonic-Sensi-Agar ® der Firma E. Merck (Art.-Nr. 16318, Nährboden Handbuch - 100 Jahre Nährböden Merck, Firma Merck KGaA, 64271 Darmstadt, Deutschland) oder auch der ASS-Agar (Antibiotika-Sulfonamid-Sensibilitätstest-Agar) der Firma E. Merck (Art.-Nr. 5392) und andere geeignete Medien anderer Hersteller verwendet werden. Der Mueller-Hinton-Agar wurde 1941 als Kulturnährboden entwickelt (Proc. Soc. Exp. Biol. and Med. 48, 330-333).

### BEISPIEL 1

In Anlehnung an die amtliche Sammlung von Untersuchungsverfahren nach § 35 LMBG, Untersuchung von Lebensmitteln mit dem Brillantschwarz-Reduktionstest zum Nachweis von Hemmstoffen in Milch, Nr. L 01.00/11, wird das Testverfahren durchgeführt. Ein erfindungsgemäßes Testmedium wird wie folgt hergestellt:
1.Einwaage des Nährmediums: 3,8 g Mueller-Hinton-Nährboden von Oxoid, Art.-Nr. CM 337, werden in 100 ml dest. Wasser suspendiert.
2.Aufkochen zum vollständigen Lösen des Mediums.
3. Einstellen des pH-Wertes auf 8,0 mit NaOH bei etwa 45°C.
4. Autoklavieren bei 121°C +/- 1°C über 15 min.
5. Nach Abkühlung des Nährmediums auf ca. 65°C
   (a) Zugabe von 0,8 ml Pyrimethamin-Gebrauchslösung pro 100 ml Nährmedium. Die Gebrauchslösung besteht aus 5 mg Pyrimethamin-Reinsubstanz (5-[4-Chlorphenyl]-6-ethyl-2,4-pyrimidindiamin, C₁₂H₁₃ClN₄, Mol-Gew. 248,71, Smp. 238 - 242°C) gelöst in 5 ml Ethanol und 45,0 ml Aqua dest. Die Pyrimethamin-Gebrauchslösung wird autoklaviert oder sterilisiert;
   (b) Zugabe von 4,5 ml Brillantschwarz-Gebrauchslösung pro 100 ml Testmedien. Die Brillantschwarz-Gebrauchslösung besteht aus 2 mg Brillantschwarz pro ml Aqua dest. Diese Lösung wird autoklaviert.
   (c) Zugabe einer Sporensuspension von Bacillus stearothermophilus var. calidolactis, Stamm C 953 in Mengen, die einen Inhalt von ca. 5 x 10⁶ bis 2 x 10⁷ Sporen/ml Testmedium gewährleisten.
   (d) Zugabe von 1 ml EGTA-Gebrauchslösung pro 100 ml Nährmedium. Die EGTA-Gebrauchslösung besteht aus 50 mg Ethylenglykol-bis-(β-aminoethylether)-N,N-tetraessigsäure pro ml Aqua dest. Diese Lösung wird autoklaviert.
6. Abfüllen von jeweils 100 µl des erfindungsgemäßen Testmediums in jede Kavität einer Mikrotiterplatte (96 Lochplatte, z.B. von Nunc).

Für dieses Testmedium und die Vergleichsvarianten wurde mit einer homogenisierten pasteurisierten Vollmilch die Nachweisgrenze für folgende Tierarzneimittelrückstände bestimmt:
Penicillin G
Sulfamethazin
Sulfathiazol
und die Inkubationszeiten gemessen. Zum Vergleich wurde ein erster Test mit einem Testmedium ohne Pyrimethamin und ohne EGTA, ein zweiter Test mit einem Testmedium unter Zugabe von Pyrimethamin aber ohne EGTA und ein dritter Test mit Zugabe von EGTA aber ohne Pyrimethamin durchgeführt.

Die Testergebnisse sind nachstehend in Tabelle 1 zusammengefaßt:

**Tabelle 1**

| | Penicillin G | Sulfamethazin | Sulfathiazol | Inkubations zeit in Std. |
|---|---|---|---|---|
| Mueller-Hinton Agar | 1 - 2 | > 5000 | > 5000 | 3.30 - 4.00 |
| Mueller-Hinton Agar mit Pyrime<-> | 1 - 2 | 100 | 50 | 3.45 - 4.15 |
| thamin Mueller-Hinton Agar mit EGTA | 1 2 | > 5000 | > 5000 | 2.30 - 3.00 |
| Mueller-Hinton-Agar mit Pyrimemin/EGTA | 1 - 2 | 100 | 50 | 2.45 - 3.15 |
| Angaben in ng Reinsubstanz/ml Milch | | | | |

Die Tabelle zeigt, daß die niedrigen Nachweisgrenzen für Penicillin und die Erfassung der amtlichen Höchstmengen für Sulfonamide nach Zugabe von Pyrimethamin unabhängig sind von der Zugabe von EGTA, daß durch die Zugabe von ETGA aber die Inkubationszeit um etwa 1 h gesenkt werden konnte, so daß die max. Inkubationszeit von 2.45 - 3.15 h das gesetzte Ziel einer weitgehenden kurzen Gesamttestzeit zum Nachweis von Penicillinen und Sulfonamiden in einem Test voll erreicht wurde.

### BEISPIEL 2

Herstellung eines erfindungsgemäßen Testmediums entsprechend Beispiel 1.

Für dieses Testmedium und die Vergleichsvarianten wurde mit einer homogenisierten pasteurisierten Vollmilch die Nachweisgrenze für folgende Tierarzneimittelrückstände bestimmt:
Penicillin G
Sulfamethazin
Oxytetracylin
und die Inkubationszeiten gemessen. Zum Vergleich wurde ein erster Test erneut mit einem Testmedium ohne Zugabe von Pyrimethamin und EGTA durchgeführt.

In einem zweiten Test war Pyrimethamin ohne EGTA vorhanden. In einem dritten Test (Variante A) war Pyrimethamin entsprechend Beispiel 1 vorhanden, aber abweichend von Beispiel 1 wurde kein EGTA in das Testmedium eingebracht, sondern zu jeder Kavität, die bereits 100 µl einer Milchprobe enthielt, wurden 24 µl der EGTA-Gebrauchslösung einpipettiert.

In einem vierten Test (Variante B) waren Pyrimethamin und EGTA entsprechend Beispiel 1 in dem Testmedium enthalten und in einem fünften Test (Variante C) wurden zusätzlich zur Variante B noch zu jeder Kavität die bereits 100 µl einer Milchprobe enthielt, wurden 10 µl der EGTA-Gebrauchslösung einpipettiert.

Die Testergebnisse sind nachstehend in Tabelle 2 zusammengefaßt:

**Tabelle 2**

| | Penicillin G | Sulfamethazin | Oxytetracyclin | Inkubations zeit in Std. |
|---|---|---|---|---|
| Mueller-Hinton Agar | 1 - 2 | > 5000 | 800-1000 | 3.30 - 4.00 |
| Mueller-Hinton Agar mit Pyrimethamin | 1 - 2 | 100 | 800-1000 | 3.45 - 4.15 |
| Variante A Mueller-Hinton mit Pyrimethamin + EGTA in Probe | 1 - 2 | 150-250 | 100-200 | 2.45 - 3.15 |
| Variante B Mueller-Hinton mit Pyrimethamin/EGTA | 1 - 2 | 100 | 800 | 2.45 - 3.15 |
| Variante C Mueller-Hinton mit Pyrimethamin/EGTA + EGTA in Probe | 1 - 2 | 150-250 | 150-200 | 2.45 - 3.15 |
| Angaben in ng Reinsubstanz/ml Milch | | | | |

Überraschenderweise ergab sich aus Tabelle 2, daß EGTA ein wesentlicher Hemmstoff-Wirkungsverstärker für Tetracycline ist, wobei die besten Ergebnisse erzielt wurden, wenn EGTA nur in der zu untersuchenden Probe (Variante A) enthalten ist. Nur geringfügig schlechtere Ergebnisse wurden erzielt, wenn EGTA auch noch dem Medium zugegeben wurde. In allen Fällen, Varianten A bis C, wurden befriedigende kurze Inkubationszeiten erhalten. Die niedrige Nachweisgrenze für Penicillin blieb durch die Zugabe von EGTA im Medium und/oder in der zu untersuchenden Probe unberührt. Lediglich bei Sulfamethazin ist die Nachweisgrenze durch EGTA in der Milch (Variante A und Variante C) etwas verschlechtert.

Ein vergleichbar guter Tetracyclinnachweis bei einem pH-Wert des Testmediums von 8.0 ist bisher nicht bekannt geworden. Bisher konnte der Nachweis von Tetracyclinen nur verbessert werden, wenn der pH auf einen Wert von 6.0 eingestellt war. Bei einem solchen pH-Wert verschlechtern sich aber gleichzeitig die Nachweisgrenzen für verschiedene andere Antibiotika und Sulfonamide. Es ist zu vermuten, daß einen wesentlichen Einfluß auf die verbesserten Nachweisgrenzen für Tetracycline bei einem pH von 8.0 auch das erfindungsgemäß verwendete Nährmedium hat.

Es ist schon vorgeschlagen worden, für Tetracycline die Nachweisgrenze durch Zugabe von Ammoniumoxalat zu senken (Suhren G., Heeschen W., Milchwissenschaft 45(6) 1990, S. 343-347). Diese Methode führte zwar zu einem verbesserten Nachweis der Tetracycline in verschiedenen kommerziellen Testsystemen, dabei wurde die Inkubationszeit aber um 0,5 bis 0.75 h verlängert. Ein wesentlicher Nachteil dieser Methode besteht vor allem darin, daß neben dem Calcium auch die für das Wachstum des Testkeims essentiellen Mineralien Magnesium, Kalium usw. gebunden werden.

Demgegenüber war es überraschend, daß in der Milch die Chelatbindungen der Tetracycline mit Calcium und anderen Metallionen durch EGTA im ausreichenden Maß aufgehoben werden können, so daß die Tetracycline ihre antibiotische Wirkung zurückerhielten. Weiterhin war es überraschend, daß EGTA außerdem positiv auf die Germination (Auskeimung) der Sporen wirkte. Die Sporen keimten auch bei suboptimalen Nährmedien gleichzeitig aus. Dies hängt vermutlich damit zusammen, daß der stark calciumhaltige Sporenmantel vom EGTA angegriffen wird und dies bereits als Anreiz für die Sporen ausreicht, um in die Phase der Germination überzugehen.

Zur Verbesserung der Nachweisgrenze der Tetracycline kann (ohne das Wachstum der Testkeime zu beeinträchtigen) dem zu untersuchenden Probenmaterial EGTA aximal in der äquimolaren Menge des freien Calciums des Probenmaterials zugegeben werden (Variante A).

Je nach dem verwendeten Medium und dem zu untersuchenden Probenmaterial können die wirksamen Kcnzentrationen des EGTA als Hemmstoffverstärker und als Germinationsbeschleuniger (-anreger) variieren. Wird die maximale Menge des EGTA überschritten, verlängert sich die Testzeit. Bei Milch liegt der Bereich zwischen etwa 2000 µg und 100 µg EGTA pro 100 µl Milch. Dabei wird EGTA vorzugsweise in dem Bereich 500 µg bis 1500 µg pro 100 µl Milch eingesetzt. Mit gutem Erfolg wurden 1200 µg EGTA pro 100 µl Milch verwendet. Dabei kann es von Vorteil sein, EGTA der Milch gepuffert zuzugeben, um einer Erniedrigung des pH-Wertes entgegenzuwirken.

Wird EGTA nicht dem zu untersuchenden Material, sondern allein dem Testmedium zugesetzt (Variante B), so sind die EGTA-Konzentrationen deutlich geringer anzusetzen. Bei Milch als Testmaterial und einer Sporenzahl von 5 x 10⁶ bis 2 x 10⁷ Sporen/ml Testmedium liegt der EGTA-Bereich zwischen 50 µg und 2500 µg/ml Testmedium, vorzugsweise liegt der Bereich zwischen 100 µg und 1500 µg/ml Testmedien. Mit gutem Erfolg wurden 500 µg EGTA pro ml Testmedium verwendet.

Der Komplexbildner EGTA kann auch kombiniert im zu untersuchenden Material und im Testmedium verwendet werden (Variante C). Die Testzeit wird bei einer Verwendung von EGTA im zu untersuchenden Material nur unwesentlich (ca. 5 bis 10 min) verlängert oder wird bei suboptimalen Testmedien sogar verkürzt. Bei Verwendung von EGTA nur im Testmedium wird die Testzeit wesentlich beschleunigt. Bei den Varianten A und B im zu untersuchenden Material und im Medium wird die visuelle Ablesbarkeit des Testergebnisses verbessert oder nicht negativ beeinflußt.

Vergleichbar gute Ergebnisse als Hemmstoff-Wirkungsverstärker und als Germinationsbeschleuniger sind erfindungsgemäß auch mit den vorstehend genannten Komplexbildnern DTPA, CDTA, EDTA, NTA, TTHA und mit N-(2-Hydroxyethyl)-ethylendiamin-N,N,N-triessigsäure zu erwarten.

Durch den Einsatz von EGTA in der Milch wird lediglich der Nachweis von Sulfonamiden etwas verschlechtert. Auf die übrigen getesteten Hemmstoffe hat das EGTA keine meßbaren negativen Einflüsse. Dank einer erfindungsgemäßen Verwendung von Pyrimethamin und dem standardisierten Mueller-Hinton-Nährmedium für den Bacillus stearothermophilus var. calidolactis war aber der negative Einfluß von EGTA auf den Nachweis von Sulfonamiden nur relativ gering.

Die Nachweisgrenze der Hemmstoffe in den zu untersuchenden Proben kann auch dadurch verbessert werden, daß die visuelle Ablesbarkeit der Testergebnisse durch den Indikator-Farbumschlag verbessert wird.

Bisher wurden dem Testmedium lediglich ein Indikator zugegeben. Hierbei wurde bisher zwischen einem Redox-Indikator oder einem pH-Indikator gewählt.

Die Testorganismen werden bei hohen Konzentrationen von Antibiotika und Sulfonamiden vollständig gehemmt. Mit sinkender Konzentration erfolgt eine zunehmend unvollständige Hemmung. Die Testkeime können in diesen Bereichen nur im geringen Umfang oder verlangsamt wachsen. Dabei reduzieren die Keime den Farbstoff nur teilweise oder es entstehen Stoffwechselprodukte, die den pH-Wert des Mediums verändern. Es entsteht ein Übergangsbereich mit Farbtönen, die sich nicht eindeutig von der Positivkontrolle abgenzen lassen. Dieser Bereich kann zwar mit erheblichem Aufwand durch photometrische Messungen genauer abgegrenzt werden, was allein visuell nicht oder nur fehlerhaft möglich ist.

Um diese Schwierigkeiten zu umgehen und die visuelle Ablesbarkeit deutlich zu verbessern, werden erfindungsgemäß mindestens zwei Indikatoren mit unterschiedlichen Wirkungsprinzipien verwendet. Zum Beispiel kann es sich um einen Redox-Indikator und einen pH-Indikator handeln. Beide Indikatoren dürfen sich nicht gegenseitig beeinflussen und ihre Farben sollen sich unabhängig voneinander verändern. Dabei wird die Inkubationszeit nicht verlängert. Die durch die unterschiedlichen Wirkungsprinzipien erhaltenen Farbtöne in dem genannten Übergangsbereich "addieren" sich lediglich, so daß die Farbintensität insbesondere bei einer unvollständigen Hemmung wesentlich gesteigert werden kann. Die Farbtöne des Übergangsbereichs lassen sich mit zwei Indikatoren deutlicher von der Negativkontrolle unterscheiden, als wenn, wie bisher, nur ein Indikator verwendet wird.

Grundsätzlich können erfindungsgemäß alle Arten von Indikatoren mit unterschiedlichen Wirkungsprinzipien in Frage kommen, wobei mindestens zwei Indikatoren mit unterschiedlichen Wirkungsmechanismen vorhanden sein müssen.

Dabei können auch mehrere Indikatoren mit gleichen Wirkungsmechanismus gleichzeitig verwendet werden. Die Mengen der einzelnen Indikatoren bzw. die Wahl der pH-Bereiche müssen aufeinander eingestellt sein, damit eine gemeinsame Entfärbungszeit gegeben ist. Nur bei im wesentlichen zeitgleichen Farbumschlägen beider Indikatoren wird ein verbesserter visueller Effekt erreicht.

Bei optimaler Einstellung der Verhältnisse zwischen den Indikatoren wird die Testzeit nicht beeinflußt. Die erfindungsgemäße kombinierte Verwendung wenigstens zweier Indikatoren mit unterschiedlichen Wirkungsmechanismen verbessert für alle Substanzen die Nachweisgrenzen, wie das nachstehende Beispiel 3 zeigt, in dem als Indikatoren Billantschwarz und Bromkresolpurpur verwendet werden.

### Beispiel 3

Herstellung eines erfindungsgemäßen Testmediums:
1. Einwaage des Nährmediums: 3,8 g Mueller-Hinton-Nährboden von Oxoid, Art.-Nr. CM 337 werden in 100 ml dest. Wasser suspendiert.
2. Aufkochen zum vollständigen Lösen des Mediums.
3. Einstellen des pH-Wertes auf 8,0 mit NaOH bei etwa 45°C.
4. Autoklavieren bei 121°C +/- 1°C über 15 min.
5. Nach Abkühlung des Nährmediums auf ca. 65°C
   (a) Zugabe von 0,8 ml Pyrimethamin-Gebrauchslösung pro 100 ml Nährmedium. Die Gebrauchslösung besteht aus 5 mg Pyrimethamin-Reinsubstanz (5-[4-Chlorphenyl]-6-ethyl-2,4-pyrimidindiamin, C₁₂H₁₃ClN₄, Mol-Gew. 248,71, Smp. 238 - 242°C) gelöst in 5 ml Ethanol. und 45,0 ml Aqua dest. Die Pyrimethamin-Gebrauchslösung wird autoklaviert oder sterilisiert;
   (b) Zugabe von 4,5 ml Brillantschwarz-Gebrauchslösung pro 100 ml Testmedium. Die Brillantschwarz-Gebrauchslösung besteht aus 2 mg Brillantschwarz pro ml Aqua dest. Diese Lösung wird autoklaviert.
   (c) Zugabe einer Sporensuspension von Bacillus stearothermophilus var. calidolactis, Stamm C 953 in Mengen, die einen Inhalt von ca. 5 x 10⁶ bis 2 x 10⁷ Sporen/ml Testmedium gewährleisten.
   (d) Zugabe von 1 ml EGTA-Gebrauchslösung pro 100 ml Nährmedium. Die EGTA-Gebrauchslösung besteht aus 50 mg Ethylenglykol-bis-(β-aminoethylether)-N,N-tetraessigsäure pro ml Aqua dest. Diese Lösung wird autoklaviert.
   (e) Bromkresolpurpurgebrauchslösung: 1 mg/ml H₂O, steril; 3 ml Gebrauchslösung/100 ml Medium
6. Abfüllen von jeweils 100 µl des erfindungsgemäßen Testmediums in jede Kavität einer Mikrotiterplatte (96 Lochplatte, z. B. von Nunc).
Nachweisgrenzen wurden mit pasteuerisierter homogenisierter Vollmilch ermittelt. Die Testergebnisse sind nachstehend in Tabelle 3 zusammengefaßt:

**Tabelle 3**

| | Penicillin G | Cloxacillin | Sulfamethazin | Inkubationszeit in Std. |
|---|---|---|---|---|
| Mueller-Hinton Agar mit Brillantschwarz | 1 - 2 | 10 - 20 | 100 | 2.45 - 3.15 |
| Mueller-Hinton Agar mit Bromkresolpurpur | 1 - 2 | 10 - 20 | 100 | 2.45 - 3.15 |
| Mueller-Hinton Agar mit Brillantschwarz und Bromkresolpurpur | 1 | 10 | 50 | 2.45 - 3.15 |
| Angaben in ng Reinsubstanz/ml Milch | | | | |

Die Tabelle zeigt, daß die Nachweisgrenzen bei Verwendung von 2 Indikatoren (Brillantschwarz und Bromkrsolpurpur) mit unterschiedlichen Wirkungsprinzipen nochmals wesentlich gesenkt werden konnten, ohne daß die Inkubationszeit verlängert wird.

## Patentansprüche

1. Testmedium für einen mikrobiologischen Hemmstofftest, insbesondere zum Nachweis von Hemmstoffen (Tierarzneimittelrückständen) in Produkten tierischen Ursprungs, wobei das Testmedium einen Testorganismus (Testkeim), ein Nährmedium für den Testkeim und gegebenenfalls ein optisches Anzeigemittel (Indikator) enthält, das durch den im Nährmedium wachsenden Testkeim einen Farbumschlag erfährt, wenn er nicht durch einen Hemmstoff am Wachstum gehemmt wird,
**dadurch gekennzeichnet**, daß
das Testmedium wenigstens ein Mittel zur Erhöhung der Testempfindlichkeit enthält, das aus der nachstehenden Gruppe ausgewählt ist:
a) wenigstens einen Komplexbildner mit einer hohen Kapazität und Selektivität für die Komplexierung von Metallionen, die die Wirkung von Tierarzneimitteln und/oder die Keimung der Sporen beeinträchtigen,
b) Pyrimethamin in Verbindung mit Bacillus stearothermophilus var. calidolactis als Testkeim, und
c) als optisches Anzeigemittel die Kombination wenigstens zweier Indikatoren mit unterschiedlichen Wirkungsprinzipien.

2. Testmedium nach Anspruch 1, dadurch gekennzeichnet, daß der Komplexbildner eine hohe Bindungskapazität für die Komplexierung von Metallionen, insbesondere 2-wertige Metallionen aufweist.

3. Testmedium nach Anspruch 1, dadurch gekennzeichnet, daß der Komplexbildner Bis(aminoethyl)-glycolether-N,N,N',N'-tetraessigsäure (C₁₄H₂₄N₂O₁₀, M = 380,35 g/Mol, abgekürzt EGTA) ist.

4. Testmedium nach Anspruch 2 oder 3, dadurch gekennzeichnet, daß der Komplexbildner Hemmstoff-Wirkungsverstärker für durch Calciumkomplexe in ihre antibiotischen Wirkung beeinträchtigte Tetracycline und Sporenkeimungsförderer (Germinationsbeschleuniger) für an einer Sporenkeimung gehinderte Sporen des Testkeimes ist.

5. Testmedium nach Anspruch 1, dadurch gekennzeichnet, daß das Nährmedium frei oder weitgehend frei ist von Sulfonamid-und Pyrimethamin-Antagonisten.

6. Testmedium nach Anspruch 1, dadurch gekennzeichnet, daß das Testmedium standardisierten Mueller-Hinton-Agar als Nährmedium für den Testkeim enthält.

7. Testmedium nach Anspruch 1, dadurch gekennzeichnet, daß das optische Anzeigemittel aus der Kombination wenigstens eines Redoxindikators und wenigstens eines pH-Indikators besteht.

8. Testmedium nach Anspruch 7, dadurch gekennzeichnet, daß der Redoxindikator Brillantschwarz und der pH-Indikator Bromkresolpurpur ist.

9. Verfahren zum Nachweis von Hemmstoffen mit einem Testmedium nach Anspruch 1, dadurch gekennzeichnet, daß der zu untersuchenden Probe wenigstens ein Komplexbildner zugegeben wird.

10. Verfahren nach Anspruch 9, dadurch gekennzeichnet, daß ein gepufferter Komplexbildner in der zu untersuchenden Probe verwendet wird.

11. Verfahren nach Anspruch 9, dadurch gekennzeichnet, daß der Komplexbildner als Hemmstoff-Wirkungsverstärker für Tetracycline und/oder als Sporenkeimungsförderer (Germinationsbeschleuniger) verwendet wird.

12. Verfahren nach einem der vorstehenden Ansprüche zur Untersuchung von Milchproben auf Tierarzneimittelrückstände.

## Claims

1. Test medium for a microbiological growth inhibitor test, especially for demonstrating the presence of growth inhibitors (veterinary pharmaceutical preparation residues) in products of animal origin, wherein the test medium contains a test organism (test germ), a nutrient medium for the test germ and optionally an optical indicating agent (indicator), which experiences a colour change from the test germ growing in the nutrient medium, when it is not inhibited from growing by a growth inhibitor, characterized in that the test medium contains at least one medium for increasing the test sensitivity, selected from the following group:
a) at least one complex former with a high capacity and selectivity for complexing metal ions, which affect the effect of veterinary pharmaceutical preparations and/or the germination of the spores,
b) pyrimethamine in combination with bacillus stearothermophilus var. calidolactis as test germ, and
c) the combination of at least two indicators with different working principles as optical indicating agent.

2. Test medium according to claim 1, characterized in that the complex former has a high bonding capacity for the complexing of metal ions, especially divalent metal ions.

3. Test medium according to claim 1, characterized in that the complex former is bis (aminoethyl) -glycolether- N,N,N',N' -tetraacetic acid (C₁₄H₂₄N₂O₁₀, M = 380.35 g/mole. abbreviated EGTA).

4. Test medium according to claim 2 or 3, characterized in that the complex former is growth inhibitor intensifier for tetracyline affected in its antibiotic action by calcium complexes and spore germination promoters (germination accelerators) for spores of the test germ prevented from spore germination.

5. Test medium according to claim 1, characterized in that the nutrient medium is free or largely free from sulphonamide and pyrimethamine competitive inhibitors.

6. Test medium according to claim 1, characterized in that the test medium contains standardised Mueller-Hinton agar as nutrient medium for the test germ.

7. Test medium according to claim 1, characterized in that the optical indicating agent consists of the combination of least one redox indicator and at least one pH indicator.

8. Test medium according to claim 7, characterized in that the redox indicator is brilliant black and the pH indicator is bromo-cresol purple.

9. A method of demonstrating the present of growth inhibitors with a test medium according to claim 1, characterized in that the at least one complex former is added to the sample to be investigated.

10. A method according to claim 9, characterized in that a buffered complex former is used in the sample to be investigated.

11. A method according to claim 9, characterized in that the complex former is used as a growth inhibitor intensifier for tetracycline and/or as a spore germination promoter (germination accelerator).

12. A method according to any of the preceding claims for investigating milk samples for veterinary pharmaceutical preparation residues.

## Revendications

1. Milieu de test pour un test microbiologique de substances inhibitrices, notamment pour la détection de substances inhibitrices (résidus de médicaments vétérinaires) dans des produits d'origine animale, ledit milieu de test contenant un organisme de test (souche de test), un milieu nutritif pour la souche de test, et le cas échéant un agent de visualisation optique (indicateur) virant de couleur par suite de la croissance de la souche de test dans le milieu nutritif lorsque la croissance n'est pas inhibée par une substance inhibitrice, caractérisé en ce que le milieu de test contient au moins un agent pour augmenter la sensibilité du test, choisi dans le groupe ci-dessous :
a) au moins un agent complexant avec une capacité et une sélectivité élevées pour la complexation d'ions métalliques qui contrarient l'action des médicaments vétérinaires et/ou la germination des spores,
b) de la pyriméthamine associée à *Bacillus stearothermophilus* var. *calidolactis* comme souche de test, et
c) comme agent de visualisation optique, l'association d'au moins deux indicateurs ayant des principes d'action différents.

2. Milieu de test selon la revendication 1, caractérisé en ce que l'agent complexant présente une capacité de liaison élevée pour la complexation d'ions métalliques, notamment d'ions métalliques divalents.

3. Milieu de test selon la revendication 1, caractérisé en ce que l'agent complexant est l'acide bis(aminoéthyl)-glycoléther-N,N,N',N'-tétracétique (C₁₄H₂₄N₂O₁₀, M = 380,35 g/mol, en abrégé EGTA).

4. Milieu de test selon la revendication 2 ou la revendication 3, caractérisé en ce que l'agent complexant est un renforçateur de l'effet de substance inhibitrice pour des tétracyclines dont l'activité antibiotique est contrariée par des complexes de calcium et un promoteur de germination des spores (accélérateur de germination) pour des spores de la souche de test dont la germination des spores est empêchée.

5. Milieu de test selon la revendication 1, caractérisé en ce que le milieu nutritif est exempt ou essentiellement exempt d'antagonistes des sulfamides et de la pyriméthamine.

6. Milieu de test selon la revendication 1, caractérisé en ce que le milieu de test contient du milieu gélosé Mueller-Hinton standardisé comme milieu nutritif pour la souche de test.

7. Milieu de test selon la revendication 1, caractérisé en ce que l'agent de visualisation optique se compose de l'association d'au moins un indicateur d'oxydoréduction et d'au moins un indicateur de pH.

8. Milieu de test selon la revendication 7, caractérisé en ce que l'indicateur d'oxydoréduction est le noir brillant et l'indicateur de pH est le pourpre de bromocrésol.

9. Procédé de détection de substances inhibitrices avec un milieu de test selon la revendication 1, caractérisé en ce que l'on ajoute au moins un agent complexant à l'échantillon à analyser.

10. Procédé selon la revendication 9, caractérisé en ce que l'on utilise un agent complexant tamponné dans l'échantillon à analyser.

11. Procédé selon la revendication 9, caractérisé en ce que l'agent complexant est utilisé comme renforçateur de l'effet de substance inhibitrice pour des tétracyclines et/ou comme promoteur de germination des spores (accélérateur de germination).

12. Procédé selon l'une des revendications précédentes pour la recherche de résidus de médicaments vétérinaires dans des échantillons de lait.
